# EUROPEAN PATENT APPLICATION

(11) **EP 3 777 925 A1**
(43) Date of publication of application: **17.02.2021**
(21) Application number: 18925966.6
(22) Date of filing: 13.07.2018
(51) Int. Cl.: A61M 5/158

(54) **FLUID COLLECTION INJECTION NEEDLE**

(71) Applicant: Hundred Co., Ltd., Fukushima 963-0201 (JP)
(72) Inventor: KAYAMOTO, Naoyuki, Koriyama-shi, Fukushima 963-0201 (JP); OKADA, Yuki, San Rafael, California 94901 (US)
(74) Representative: Geskes, Christoph
(86) International application number: PCT/JP2018/026612
(87) International publication number: WO 2020/012664

(57) **Abstract**

A liquid collection-injection needle assembly is provided including a needle cap that accommodates and protects a needle, and that is designed to be used for other purposes as well, so that the needle cap is effectively used, and the number of parts required for assembly is reduced. The assembly includes a needle holding member 2 that holds a needle 4 after needle 4 has been inserted into holding member 2, and a needle cap 3 that is configured to be connected detachably to a front part of needle holding member 2, and to protect the exposed portion of needle 4 that is exposed from the needle holding member after needle 4 has been inserted into needle holding member 2. In the state where needle cap 3 communicates with needle holding member 2, an end portion of the needle cap is capable of communicating with a rear part of needle holding member 2.

## Description

### Technical Field

The present invention relates to a liquid collection-injection needle assembly, to which various medical devices are connectable.

### Background Art

A liquid collection-injection needle assembly that can be used for both blood collection and chemical injection is disclosed, for example, in Japanese Unexamined Patent Application Publication No. 2017-184985. In such a liquid collection-injection needle assembly, the needle is held in such a way that the distal portion is exposed relative to the needle holding member that has a flap part. In this structure, the liquid collection-injection needle assembly includes a cap to accommodate the exposed needle portion in order to protect the needle and to prevent the needle from accidentally sticking a worker. The needle cap covers the exposed needle portion while the needle is inserted into the cap, and the rear-end portion of the cap is attached to the tip of the needle holding member with the rear-end portion being inserted into the tip of the needle holding member. Such a structure to attach the needle cap to the needle holding member is disclosed in Patent Document 1 (Japanese Unexamined Utility Model (Registration) Application Publication No. H05-41558), and Patent Document 2 (Japanese Utility Model Registration No. 3016719).

Patent Document 1 provides a structure, in which a fitting cylindrical part, which is the tip portion of a needle holding member, is provided with mating projections while the rear-end portion of a needle cap is provided with mating grooves. The needle cap is stably attached to the fitting cylindrical part by fitting the mating projections into the mating grooves.

Patent Document 2 provides a structure that includes a needle cap to a flap part provided to a cylindrical holding part, in which the needle cap is provided with a horizontal cutout part, and the flap part is folded to be inserted into the cutout part, whereby the flap part is stably engaged with the needle cap.

### Prior-art Document

### Patent Document

Patent Document 1: Japanese Unexamined Utility Model (Registration)
   Application Publication No. H05-41558
Patent Document 2: Japanese Utility Model Registration No. 3016719

### Summary of the Invention

### Problems to be Solved by the Invention

Conventionally, a needle cap has only been used to cover and protect a needle, so that after a liquid collection-injection needle assembly is taken out from a packaging bag, the needle cap is removed from the needle and discarded. Therefore, the needle cap has not been used to collect blood or inject chemicals, which results in a waste of resources.

Also, in order to connect the liquid collection-injection needle assembly to an external medical device such as a hematocrit tube or a syringe, an intermediate component such as a connecting tube needs to be set separately in advance on the liquid collection-injection needle assembly, so that the needle is connected to the external medical device via an intermediate component. This results in an increase in the number of components of the liquid collection-injection needle assembly, which makes the needle assembly complicated to handle and also expensive.

The present invention is made to address those conventional problems, and the objective of the invention is to provide a liquid collection-injection needle assembly to which various medical devices can be connected. Another objective of the present invention is to provide a liquid collection-injection needle assembly configured such that the needle cap to protect the needle can be used to connect the needle holding member to external medical devices, which enables effective use of the needle cap.

### Means for Solving Problems

According to an embodiment of the present invention, a liquid collection-injection needle assembly includes a needle, a needle holding member, and a needle cap. The needle holding member includes a holding cylindrical part that holds the needle after a needle base of the needle has been inserted into the holding cylindrical part, and a connecting cylindrical part that is formed so as to communicate with the holding cylindrical part. The needle cap is formed into a tubular shape so as to cover the exposed portion of the needle, in which the exposed portion is exposed from the holding cylindrical part, and is configured to be connected detachably to the needle holding member. The needle cap is configured to be directly or indirectly connected to the connecting cylindrical part, so that the needle cap is capable of communicating with the connecting cylindrical part.

According to another feature of the present invention, the connecting cylindrical part and the needle cap form an engaging structure by which they are mutually engaged and detached.

According to yet another feature of the present invention, the needle cap is provided with a structure for connection that allows the needle cap being in a state connected to the connecting cylindrical part to be connected to an external medical device.

According to another feature of the present invention, the engaging structure is configured such that the front end of the needle cap is to be inserted into the connecting cylindrical part to be mutually fitted to each other and to connect them.

According to yet another feature of the present invention, the engaging structure is configured such that at least one of the connecting cylindrical part or an end portion of the needle cap is provided with projections that allow the connecting cylindrical part and the end portion of the needle cap to engage with each other.

According to an additional feature of the present invention, the engaging structure is configured such that the connecting cylindrical part and the end portion of the needle cap are provided with a threaded member that allows the connecting cylindrical part and the end portion of the needle cap to screw together with each other.

According to yet another feature of the present invention, the engaging structure is configured such that at least either of the connecting cylindrical part and the end portion of the needle cap is provided with a rough surface that generates frictional force between the connecting cylindrical part and the end portion of the needle cap.

According to another feature of the present invention, the engaging structure is configured such that a tapered surface is formed on an inner surface of the needle cap, in which an inner diameter of the needle cap is continuously changed in an axial direction.

According to yet another feature of the present invention, the engaging structure is configured such that a stepped surface is formed on the inner surface of the needle cap, in which the inner diameter of the needle cap is changed stepwise in the axial direction.

According to an additional feature of the present invention, the engaging structure is configured such that the needle cap is formed of a flexible resin with a zigzag-shaped curved surface being formed on the inner surface of the needle cap.

According to another feature of the present invention, the needle holding member is provided with a fitting cylindrical part to which the needle cap is attached detachably, in which the fitting cylindrical part communicates with the holding cylindrical part.

According to yet another feature of the present invention, a connecting pipe is connected to the connecting cylindrical part, and the end portion of the needle cap is connected to the connecting pipe.

According to an additional embodiment of the present invention, a liquid collection-injection needle assembly includes a needle and a needle holding member. The needle holding member includes a holding cylindrical part that holds the needle after a needle base for the needle has been inserted thereinto, and a connecting cylindrical part that is formed so as to communicate with the holding cylindrical part. The connecting cylindrical part is configured to be directly or indirectly connected to an external medical device.

According to yet another feature of the present invention, the holding cylindrical part is provided with a flap part that extends in the direction intersecting with the axial direction.

### Advantageous Effects of the Invention

The present invention's needle holding member to hold a needle is provided with a connecting cylindrical part, which is configured to allow an external medical device to be connected directly or indirectly to the needle holding member, so that the present invention's needle assembly enables various types of medical devices to be connected to the needle holding member.

Also, the present invention does not waste resources because the needle cap for covering the needle can also be used in collecting blood or injecting a chemical solution. Also, because the needle cap is used to connect an external medical device to the needle holding member, there is no need to set an intermediate component such as a connecting pipe in advance to a liquid collection-injection needle assembly. Thus, the present invention provides a liquid collection-injection needle assembly having a simple structure with a reduced number of parts.

### Brief Description of the Drawings

FIGS. 1(A) and 1(B) are partially broken front views according to the First Embodiment of the present invention.
FIG. 2 is a partially broken front view showing the state in which a needle cap is connected to a connecting cylindrical part according to the First Embodiment.
FIG. 3 is an overall front view according to the First Embodiment.
FIG. 4 is a side view according to the First Embodiment.
FIG. 5 is a cross-sectional view according to the Second Embodiment.
FIG. 6 is a cross-sectional view showing the state in which a needle cap is connected to a connecting cylindrical part according to the Second Embodiment.
FIGS. 7(A), 7(B), and 7(C) are partially broken front view, partial front view, and a variation's partial front view respectively according to the Third Embodiment.
FIG. 8 is a partially broken front view according to the Fourth Embodiment.
FIG. 9 is a partially broken front view showing the second example of the Fourth Embodiment.
FIG. 10(A), 10(B) and 10(C) are partially broken front views that show sequential procedures to connect a needle cap to a connecting cylindrical part according to the Fifth Embodiment.
FIG. 11 is a partially broken front view according to the Sixth Embodiment.
FIG. 12 is a front view of a needle cap structure for connection according to the first exemplary structure of the Seventh Embodiment.
FIG. 13 is a front view of a needle cap structure for connection according to the second exemplary structure of the Seventh Embodiment.
FIGS. 14(A) and 14(B) are front views of a needle cap structure for connection according to the third exemplary structure of the Seventh Embodiment.
FIGS. 15(A) and 15(B) are cross-sectional views that show the steps of connecting a needle cap to a connecting cylindrical part according to the Eighth Embodiment.
FIG. 16 is a partially broken front view according to the Ninth Embodiment.
FIG. 17 is a partially broken front view according to the Tenth Embodiment.
FIG. 18 is a partially broken front view according to the Eleventh Embodiment.
FIG. 19 is a partially broken front view according to the Twelfth Embodiment.
FIG. 20 is a partially broken front view according to the Thirteenth Embodiment.
FIG. 21 is a partially broken front view showing a variation of the Thirteenth Embodiment.
FIG. 22 is a partially broken front view according to the Fourteenth Embodiment.
FIG. 23 is a front view according to the Fifteenth Embodiment.
FIG. 24 is a front view of an analyzer used in the Fifteenth Embodiment showing the analyzer in its separated state.

### Description of the Embodiments

Hereinafter, embodiments of the present invention will be described with reference to the accompanying drawings. In each embodiment, the same member is indicated with the same reference sign.

### First Embodiment

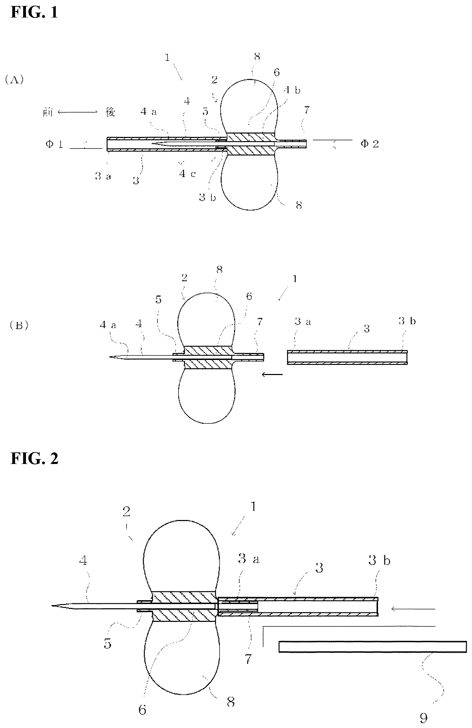

FIGS. 1 to 4 show a liquid collection-injection needle assembly 1 of the First Embodiment of the present invention. Liquid collection-injection needle assembly 1 is provided with a needle holding member 2 and a needle cap 3. Needle holding member 2 and needle cap 3 are formed entirely of resin including silicone and vinyl chloride.

Needle holding member 2 is configured to hold a needle 4. Needle 4 is connected to external medical devices via needle holding member 2. The medical devices refer to instruments to collect blood or body fluids from or to inject drug solutions to not only humans but also experimental animals such as mice. Needle 4 is formed of a pointed hollow-metal tube, through a pointed needle tip 4a of which blood or body fluids are collected from and drug solutions are injected to humans and experimental animals.

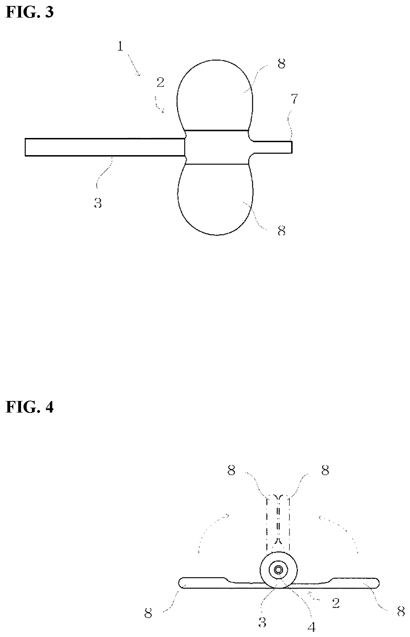

Needle holding member 2 is formed in a cylindrical shape extending in the axial direction, and includes a fitting cylindrical part 5, a holding cylindrical part 6, and a connecting cylindrical part 7. They are integrally formed so that they are respectively positioned in the front part, in the middle part, and in the rear part of needle holding member 2. Fitting cylindrical part 5, holding cylindrical part 6, and connecting cylindrical part 7 are configured to communicate with each other in the axial direction. Holding cylindrical part 6 is provided with a flap part 8 that extends in the direction intersecting with the axial direction. As shown in FIG. 4, the state of flap part 8 can be changed from the spread state shown by the solid line to the folded state shown by the chain line. In the folded state, flap part 8 is held with the thumb and forefinger, which enables a worker to use needle 4 stably. Flap part 8 shown in FIG. 4 is formed of two wings, but may be formed of one wing.

Holding cylindrical part 6 is configured to accommodate a needle base 4b of needle 4. Holding cylindrical part 6 holds needle 4 after the larger needle base 4b has been inserted into holding cylindrical part 6.

Needle cap 3 is attached to needle holding member 2 using a fitting cylindrical part 5, which extends from the front part of the holding cylindrical part, fitting cylindrical part 5 being inserted into the rear-end portion 3b of needle cap 3. Fitting cylindrical part 5 is formed such that the outer diameter of fitting cylindrical part 5 is approximately the same as the inner diameter of needle cap 3, which allows fitting cylindrical part 5 to be inserted into needle cap 3.

Connecting cylindrical part 7 is configured to be connected to needle cap 3. Connecting cylindrical part 7 is connected to needle cap 3 after connecting cylindrical part 7 has been inserted into a front end 3a of needle cap 3.

Needle cap 3 is formed of a cylinder extending in the axial direction, the cylinder having a single diameter. Needle cap 3 is provided to cover an exposed portion 4c of needle 4, the exposed portion 4c being exposed from needle holding member 2. Needle cap 3 is attached detachably to needle holding member 2 in such a way that the fitting cylindrical part 5 of needle holding member 2 is inserted into the rear-end portion 3b of needle cap 3. Covering needle 4 with needle cap 3 can protect needle 4, and can prevent needle 4 from accidentally sticking into a worker.

As shown in FIG. 1(A), needle cap 3 of this embodiment initially covers needle 4. In the case where liquid collection-injection needle assembly 1 is in use, needle cap 3 is removed from needle 4 as shown in FIG. 1(B), and is connected to the rear part of needle holding member 2 as shown in FIG. 2. That is, after connecting cylindrical part 7, which is the rear part of needle holding member 2, has been inserted into the front end 3a of needle cap 3, needle cap 3 is directly connected to needle holding member 2. In this state where needle cap 3 is connected to needle holding member 2, needle cap 3 communicates with needle holding member 2.

In the connection of needle cap 3 with connecting cylinder portion 7, it is preferable to provide an engaging structure that allows them to be mutually engaged and disengaged. The engaging structure in this embodiment, as shown in FIG. 1(A), is configured such that if the outer diameter of connecting cylindrical part 7 is Φ2, the inner diameter Φ1 of needle cap 3 is substantially equivalent to Φ2. With such an engaging structure being provided, the front end 3a of needle cap 3 can be inserted into connecting cylindrical part 7 to be mutually fitted to each other to connect them.

In addition, it is also possible to form at least either of the inner surface of the front end 3a of needle cap 3 and the outer surface of connecting cylindrical part 7 into a rough surface such as a satin-finish surface, or a knurled surface. The rough surface can generate frictional force between needle cap 3 and connecting cylindrical part 7, facilitating firm and secure connection between them.

Needle cap 3 extends behind needle holding member 2 while being connected to needle holding member 2, and a hematocrit tube 9, which is an external medical device, can be connected to this extended portion of needle cap 3. (See FIG. 2). Hematocrit tube 9 is connected to the rear-end portion 3b of needle cap 3 after tube 9 has been inserted into the rear-end portion 3b of needle cap 3. Because of this connection of hematocrit tube 9 to needle cap 3, blood and body fluids can be collected through hematocrit tube 9. In this case, because needle cap 3 has a length to be bent, the arrangement of needle cap 3 and hematocrit tube 9 can be easily adjusted so that they are in an appropriate position by bending needle cap 3.

In the above embodiment of liquid collection-injection needle assembly 1, the product of assembly 1 is shipped in a state where needle 4 is covered by needle cap 3 as shown in FIG. 1(A) with being enclosed in a packaging bag (not shown). In order for needle cap 3 to be used for connecting an external medical device to needle holding member 2, after needle assembly 1 has been taken out from the packaging bag, needle cap 3 needs to be removed from needle holding member 2, and to be connected to the connecting cylindrical part 7 of needle holding member 2. Thus, needle cap 3 can be used to connect hematocrit tube 9 to needle holding member 2.

In this embodiment, needle cap 3 can be used also for blood collection, which results in resource saving. Also, because needle cap 3 is used to connect needle holding member 2 to an external medical device such as a hematocrit tube 9, there is no need to provide an intermediate component such as a connecting pipe to the liquid collection-injection needle assembly 1 in advance. As a result, this embodiment can provide a liquid collection-injection needle assembly that has a simple structure with a reduced number of components, easy to handle assembly, and low cost to be manufactured.

### Second Embodiment

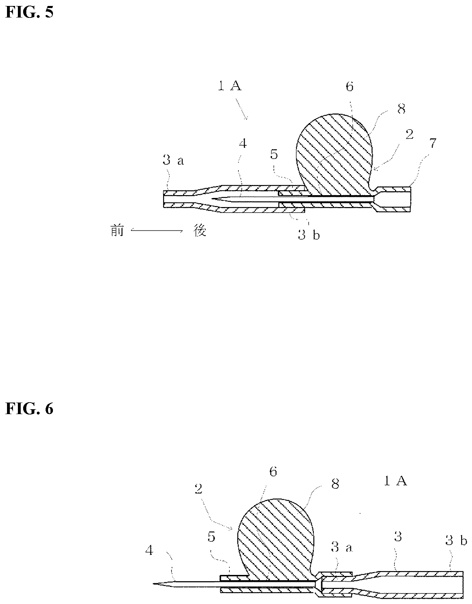

FIGS. 5 and 6 show a liquid collection-injection needle assembly 1A of the Second Embodiment of the present invention.

In this embodiment, while the front end portion 3a of a needle cap 3 is made smaller so that the diameter of front end portion 3a is reduced, the diameter of a connecting cylindrical part 7 in the rear part of needle holding member 2 is configured to be larger than the diameter of front end portion 3a of needle cap 3. This provides an engaging structure that enables the front end 3a of needle cap 3 to be inserted into connecting cylindrical part 7. The insertion of needle cap 3 into connecting cylindrical part 7 causes needle cap 3 to be fitted to connecting cylindrical part 7, which allows needle cap 3 to be connected to the rear part of needle holding member 2. Thus, this embodiment provides, similarly to the First Embodiment, liquid collection-injection needle assembly 1A that allows for effective use of needle cap 3, and that has a simple structure with a reduced number of components.

In addition, needle assembly 1A can have the opposite of this structure, in which the diameter of front end 3a of needle cap 3 is configured to be larger than that of connecting cylindrical part 7.

### Third Embodiment

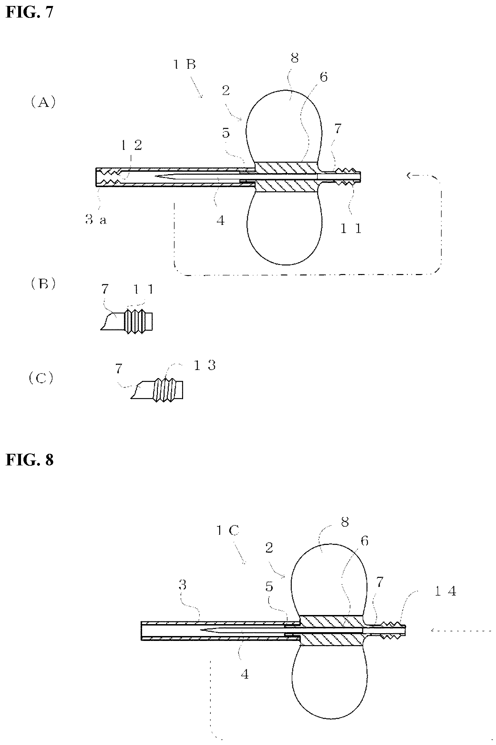

FIG. 7 shows a liquid collection-injection needle assembly 1B of the Third Embodiment of the present invention.

In this embodiment, as shown in FIGS. 7(A) and 7(B), projections 12 are formed on the inner surface of a front end 3a of a needle cap 3, and projections 11 are formed on the outer surface of a connecting cylindrical part 7, which is the rear part of a needle holding member 2. Connecting cylindrical part 7 and needle cap 3 form an engaging structure in which they are mutually engaged and detached. Removing needle cap 3 from a fitting cylindrical part 5 of needle holding member 2 and inserting the front end 3a of needle cap 3 into connecting cylindrical part 7 allows projections 11 and 12 to engage with each other, by which needle cap 3 is connected firmly to needle holding member 2. This secures the connection between needle cap 3 and needle holding member 2.

FIG. 7(C) shows the outer surface of connecting cylindrical part 7 of needle holding member 2 on which a threaded member (male threaded member) 13 is formed. The inner surface of the front end 3a of needle cap 3, to which this threaded member 13 screws, is provided with a threaded member (female threaded member). Thus, these threaded members provide an engaging structure between connecting cylindrical part 7 and needle cap 3. This kind of the engaging structure achieved by male and female threaded members allows for firm connection between connecting cylindrical part 7 (needle holding member 2) and needle cap 3.

Conversely, the engaging structure between needle cap 3 and connecting cylindrical part 7 can be achieved such that a male threaded member is formed on the inner surface of the front end 3a of needle cap 3, and a female threaded member is formed on the outer surface of connecting cylindrical part 7 of needle holding member 2.

### Fourth Embodiment

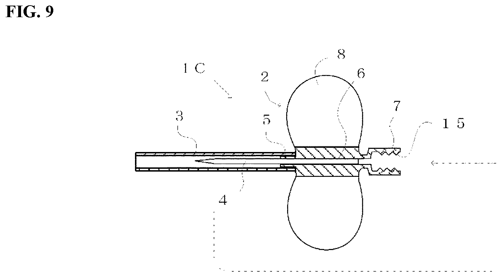

FIGS. 8 and 9 show a liquid collection-injection needle assembly 1C of the Fourth Embodiment of the present invention.

In the embodiment shown in these figures, an engaging structure is achieved by providing projections 14 and 15 to only a connecting cylindrical part 7 of a needle holding member 2. In FIG. 8, projections 14 are formed on the outer surface of connecting cylindrical part 7, and a needle cap 3, which is to be connected to connecting cylindrical part 7, is not provided with projections. Projections 14 engage with needle cap 3 after connecting cylindrical part 7 has been inserted into a front end 3a of needle cap 3.

In FIG. 9, connecting cylindrical part 7 is formed into a large-diameter cylinder to accommodate the front end 3a of needle cap 3, and projections 15 are formed on its inner surface. Needle cap 3 is not provided with projections. This structure enables the projections 15 to engage with needle cap 3 after the front end 3a of needle cap 3 has been inserted into connecting cylindrical part 7.

As shown in FIGS. 8 and 9, providing the projections 14 and 15 to connecting cylindrical part 7 allows needle cap 3 to be connected firmly to connecting cylindrical part 7 (needle holding member 2). In addition, because there is no need to provide projections 14 and 15 to needle cap 3, this embodiment has an advantage that needle cap 3 can be easily manufactured.

### Fifth Embodiment

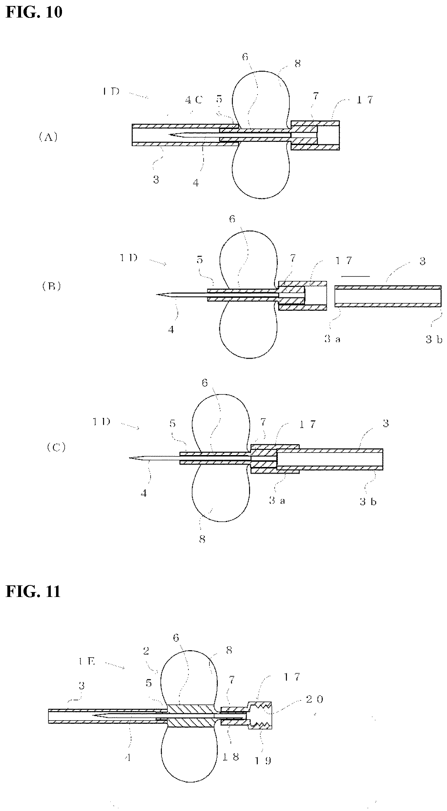

FIG. 10 shows a liquid collection-injection needle assembly ID of the Fifth Embodiment of the present invention.

This liquid collection-injection needle assembly ID is configured such that a connecting pipe 17 is connected to a connecting cylindrical part 7 in the rear part of needle holding member 2. Connecting pipe 17 is made to be tubular having a diameter into which needle cap 3 can be inserted, and after needle cap 3 has been inserted into connecting pipe 17, connecting cylindrical part 7 communicates with needle cap 3. Connecting pipe 17 is fixed to connecting cylindrical part 7 by an adhesive. Connecting pipe 17 has been provided to the existing liquid collection-injection needle assembly used to connect a hematocrit tube to the needle. In this embodiment, a product of needle assembly ID is configured such that connecting pipe 17 is preset to connecting cylindrical part 7, and needle cap 3 is attached to the front part (fitting cylindrical part 5) of needle holding member 2, needle assembly ID being enclosed in a packaging bag in this structure.

Also, in this embodiment, needle cap 3 is attached to the fitting cylindrical part 5 of needle holding member 2, whereby needle cap 3 covers the exposed portion 4c of needle 4. (See FIG. 10(A)). Removing needle cap 3 from needle holding member 2, and inserting the front end 3a of needle cap 3 into connecting pipe 17 (See FIG. 10(b)) allows needle cap 3 to be connected to connecting pipe 17. (See FIG. 10(C)). That is, in this embodiment, needle cap 3 is indirectly connected to connecting cylindrical part 7 in the rear part of needle holding member 2 via connecting pipe 17. For blood or body fluid collection, an external medical device such as a hematocrit tube is inserted into needle cap 3. The structure of this embodiment is applicable to existing needle assemblies that have a structure in which a connecting pipe 17 is preset.

### Sixth Embodiment

FIG. 11 shows a liquid collection-injection needle assembly IE of the Sixth Embodiment of the present invention.

Liquid collection-injection needle assembly IE has the same structure as that shown in FIG. 10 such that a connecting pipe 17 is connected to a connecting cylindrical part 7 in the rear part of a needle holding member 2. Connecting pipe 17 consists of an attaching portion 18, which is to be attached to connecting cylindrical part 7 by an adhesive, connecting cylindrical part 7 being inserted into attaching portion 18, and a tube receiving part 19 provided to the rear part of attaching portion 18 integrally. Tube receiving part 19 is configured to be tubular to communicate with needle holding member 2. Also, tube receiving part 19 is configured to have a large diameter, being provided with projections 20 on its inner surface.

In this embodiment, needle cap 3 is indirectly connected to needle holding member 2 through connecting pipe 17 after needle cap 3 has been inserted into tube receiving part 19 of connecting pipe 17. Because projections 20 are provided to the tube receiving part 19 of connecting pipe 17, the projections 20 engage with needle cap 3, by which needle cap 3 is tightly connected to the rear part of needle holding member 2. Thus, inserting an external medical device such as a hematocrit tube 9 into needle cap 3 allows for collection of blood samples.

### Seventh Embodiment

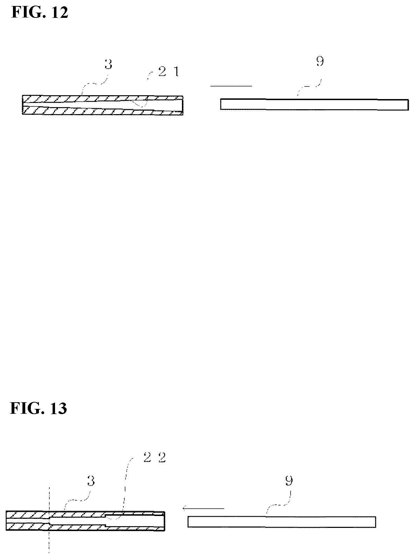

FIGS. 12 to 14 respectively show structures of a needle cap 3 used for connection with a hematocrit tube 9, which is an external medical device. The external medical device may be, for example, a syringe if applicable to those structures, in addition to hematocrit tube 9.

The cap structure shown in FIG. 12 is configured such that a tapered surface 21 is formed on the inner surface of needle cap 3, in which the inner diameter of needle cap 3 is continuously changed in the axial direction. In the case where hematocrit tube 9 is connected to needle cap 3, hematocrit tube 9 is inserted axially into needle cap 3 from the large diameter portion to the small diameter portion along the tapered surface 21 of needle cap 3, so that hematocrit tube 9 is connected to needle cap 3 at the portion of needle cap 3 where the outer diameter of hematocrit tube 9 coincides with the inner diameter of needle cap 3. Because of this structure, hematocrit tubes 9 having different outer diameters are connectable to needle cap 3.

The cap structure shown in FIG. 13 is configured such that a stepped surface 22 is formed on the inner surface of needle cap 3, in which the inner diameter of needle cap 3 is changed stepwise in the axial direction. In the case where hematocrit tube 9 is connected to needle cap 3, hematocrit tube 9 is inserted axially from the lower step part to the higher step part along stepped surface 22 of needle cap 3. In the case where hematocrit tube 9 reaches the portion of needle cap 3 where the diameter of stepped surface 22 coincides with the outer diameter of hematocrit tube 9, hematocrit tube 9 is connected to needle cap 3. Because of this structure, hematocrit tubes 9 having different outer diameters are connectable to needle cap 3.

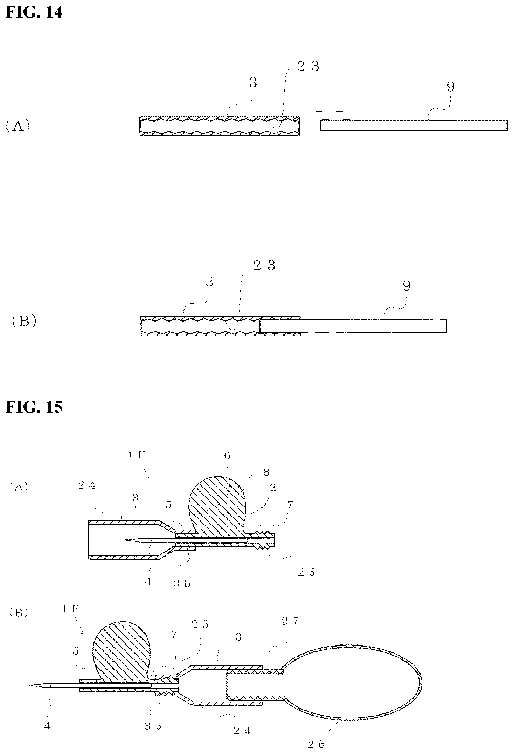

In the cap structure shown in FIG. 14, needle cap 3 is formed of a flexible resin with a zigzag-shaped curved surface 23 being provided on its inner surface. In the case where hematocrit tube 9 is connected to needle cap 3, hematocrit tube 9 is inserted into needle cap 3, so that flexible needle cap 3 expands according to the outer diameter of hematocrit tube 9, by which a frictional force is generated while zigzag-shaped curved surface 23 makes contact with hematocrit tube 9. This causes hematocrit tube 9 to stop at an appropriate insertion depth into needle cap 3, allowing hematocrit tube 9 to be connected with needle cap 3. Because of this structure, hematocrit tubes 9 having different outer diameters are connectable to needle cap 3.

### Eighth Embodiment

FIG. 15 shows a liquid collection-injection needle assembly IF of the Eighth Embodiment of the present invention.

In this embodiment, needle cap 3 is provided with an insertion part 24 having a large diameter. As shown in FIG. 15 (A), needle cap 3 is detachably connected to needle holding member 2 such that the fitting cylindrical part 5 of needle holding member 2 is inserted into the rear-end portion 3b of needle cap 3. The large-diameter insertion part 24 covers and protects this needle 4. Needle holding member 2 has a connecting cylindrical part 7 in the rear part, which is provided with projections 25.

As shown in FIG. 15(B), the needle cap 3 that has been removed from the fitting cylindrical part 5 can be attached to connecting cylindrical part 7, in which a rear-end portion 3b of needle cap 3 is attached to connecting cylindrical part 7. At this time, the projections 25 of connecting cylindrical part 7 engage with the rear-end portion 3b of needle cap 3, so that needle cap 3 can be tightly connected to connecting cylindrical part 7. In this state, an external medical device can be connected to insertion part 24 such that, for example, an insertion part 27 at the tip of a pump member 26, which is an external medical device, is inserted into the large-diameter insertion part 24 so that pump member 26 is connected to needle cap 3. Pump member 26 contains a drug solution such as an injection solution inside, so that compressing pump member 26 allows the drug solution to be injected into the affected part of a patient or an experimental animal. Accordingly, this structure can not only be applied to blood collection but also to drug administration. Also, a syringe can be connected to needle cap 3 instead of pump member 26.

In this embodiment, the needle cap 3 to cover and protect needle 4 is also used to connect pump member 26 to the needle assembly, so that needle cap 3 is effectively used without being wasted. Also, there is no need to set an intermediate part such as a connecting pipe to liquid collection-injection needle assembly IF in advance to connect pump member 26 to needle assembly IF. As a result, this embodiment can provide a liquid collection-injection needle assembly that has a simple structure with a reduced number of components, easy to handle assembly, and low cost to be manufactured.

Needle cap 3 can be connected to connecting cylindrical part 7 without providing projections 25 to connecting cylindrical part 7. Alternatively, the diameter of connecting cylinder portion 7 may be increased so that the rear-end portion 3b of needle cap 3 can be inserted into connecting cylindrical part 7.

### Ninth Embodiment

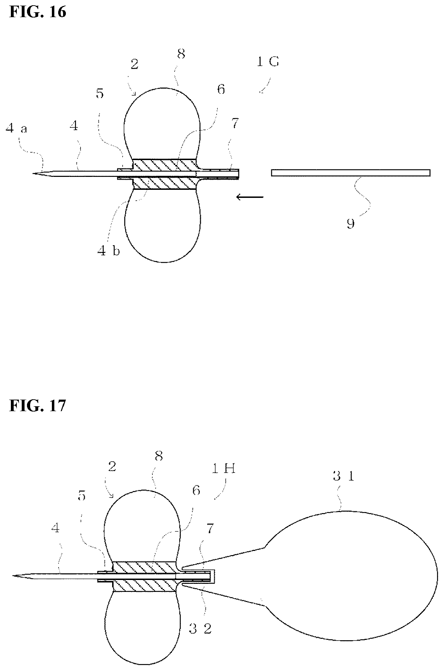

FIG. 16 shows a liquid collection-injection needle assembly 1G of the Ninth Embodiment of the present invention. The liquid collection-injection needle assembly 1G of this embodiment consists of a needle 4 and a needle holding member 2. Needle holding member 2 and a needle cap 3 are respectively formed of resin including silicone and vinyl chloride. Needle 4 and needle holding member 2 have the same structure as those in the First Embodiment described above.

Needle 4 is formed of a pointed hollow-metal tube. Collection of blood or body fluids from and injection of drug solutions to humans and experimental animals are performed through a pointed needle tip 4a of needle 4.

As in the First Embodiment, needle holding member 2 includes a fitting cylindrical part 5 positioned in the front part, a holding cylindrical part 6 in the middle part, and a connecting cylindrical part 7 in the rear part respectively, and is formed into a cylindrical shape extending in the axial direction.

Holding cylindrical part 6 is provided with a flap part 8 that extends in the direction intersecting with the axial direction. Flap part 8 is provided to be held with the thumb and forefinger for stable use of needle 4. Although flap part 8 in FIG. 16 is formed of two wings, one wing may be used as flap part 8.

Holding cylindrical part 6 is configured to accommodate a needle base 4b of needle 4. Holding cylindrical part 6 holds needle 4 after needle base 4b has been inserted into holding cylindrical part 6.

Fitting cylindrical part 5 extends from the front part of holding cylindrical part 6, so that after cylindrical part 5 has been inserted into the rear-end portion of needle cap 3 (not shown), needle cap 3 is attached to needle holding member 2. In this embodiment, because needle cap 3 is not used to connect an external medical device to needle holding member 2, needle cap 3 can be omitted from the needle assembly as appropriate. In such a case, needle holding member 2 is configured so that fitting cylindrical part 5, which is a front part of needle holding member 2, is eliminated therefrom.

In this embodiment, liquid collection-injection needle assembly 1G is configured such that a hematocrit tube 9, which is an external medical device, is directly connected to connecting cylindrical part 7. In order to connect hematocrit tube 9 to connecting cylindrical part 7, the hematocrit tube 9 to be used has an outer diameter that coincides with the inner diameter of connecting cylindrical part 7. In order to ensure and strengthen this connection, the inner surface of connecting cylindrical part 7 or the outer surface of hematocrit tube 9 may be provided with a satin-finish surface or knurled surface.

In this embodiment, hematocrit tube 9 is to be directly connected as an external medical device to connecting cylindrical part 7 of needle holding member 2 holding needle 4, which allows the collection of blood or body fluids to be performed through hematocrit tube 9. As a result, this embodiment provides a liquid collection-injection needle assembly that has a simple structure with a reduced number of components.

### Tenth Embodiment

FIG. 17 shows a liquid collection-injection needle assembly 1H of the Tenth Embodiment of the present invention. Also in this embodiment, the needle assembly is configured such that needle holding member 2 consists of a fitting cylindrical part 5, a holding cylindrical part 6, and a connecting cylindrical part 7 that communicate with each other in the axial direction, and that external medical devices are connectable to connecting cylindrical part 7.

In this embodiment, the external medical device to be used is a hollow pump member 31 that is formed of a rubber balloon. Pump member 31 and connecting cylindrical part 7 of needle holding member 2 are connected such that connecting cylindrical part 7 is inserted into a connecting port 32 in the front part of pump member 31. By inflating and deflating pump member 31 in this connected state, collection of blood or body fluids, and injection of chemical solutions can be performed through needle 4.

Also, in this embodiment, because needle cap 3 (not shown) is not used to connect an external medical device to the needle assembly, needle cap 3 can be omitted from the needle assembly as appropriate. In such a case, needle holding member 2 is configured so that fitting cylindrical part 5, which is a front part of needle holding member 2, is eliminated therefrom.

### Eleventh Embodiment

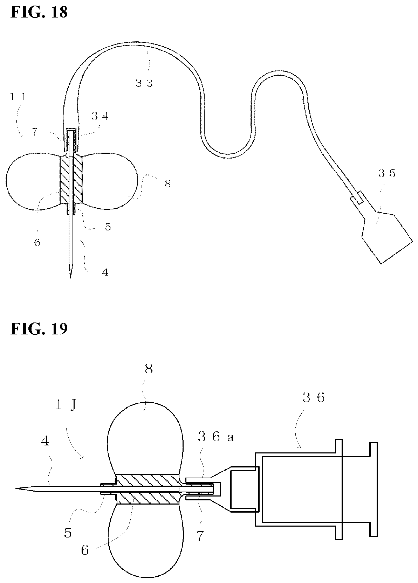

FIG. 18 shows a liquid collection-injection needle assembly 1I of the Eleventh Embodiment of the present invention.

Needle-holding member 2, which holds needle 4, consists of a fitting cylindrical part 5 positioned in the front part, a holding cylindrical part 6 in the middle part, and a connecting cylindrical part 7 in the rear part respectively, and is formed in a cylindrical shape extending in the axial direction. Connecting cylindrical part 7 allows external medical devices to be connected thereto.

In this embodiment, the external medical device is a flexible tube body 33 such as a hose. Tube body 33 and connecting cylindrical part 7 are connected such that connecting cylindrical part 7 is inserted into an insertion port 34 at the front end of tube body 33. Tube body 33 includes a liquid tube 35 such as a syringe and pump, which is attached to the rear end of tube body 33. Collection of blood or body fluid and injection of a chemical solution can be performed through a needle 4 by operating liquid tube 35.

Also in this embodiment, because needle cap 3 (not shown) is not used to connect an external medical device to the needle assembly, needle cap 3 can be omitted from the needle assembly as appropriate. In such a case, needle holding member 2 is configured so that fitting cylindrical part 5, which is a front part of needle holding member 2, is eliminated therefrom.

### Twelfth Embodiment

FIG. 19 shows a liquid collection-injection needle assembly 1J of the Twelfth Embodiment of the present invention.

A needle holding member 2, which holds needle 4, consists of a fitting cylindrical part 5 positioned in the front part, a holding cylindrical part 6 in the middle part, and a connecting cylindrical part 7 in the rear part respectively, and is formed in a cylindrical shape extending in the axial direction. Connecting cylindrical part 7 is configured to allow an external medical device to be connected thereto.

In this embodiment, the external medical device is a syringe 36. Syringe 36 and connecting cylindrical part 7 are connected such that connecting cylindrical part 7 is inserted into a syringe port 36a at the front end of syringe 36. This allows the collection of blood or body fluids and the injection of chemical solutions to be performed through needle 4.

Also in this embodiment, because needle cap 3 (not shown) is not used to connect an external medical device to the needle assembly, needle cap 3 can be omitted from the needle assembly as appropriate. In such a case, needle holding member 2 is configured so that fitting cylindrical part 5, which is a front part of needle holding member 2, is eliminated therefrom.

### Thirteenth Embodiment

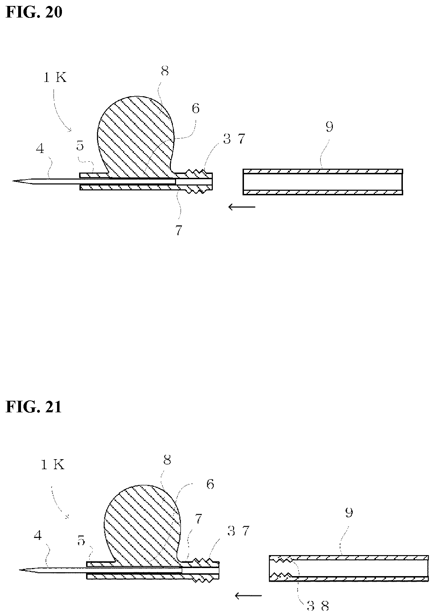

FIG. 20 shows a liquid collection-injection needle assembly 1K of the Thirteenth Embodiment of the present invention.

This embodiment has the same structure as that of Eighth Embodiment such that projections 37 are formed on the outer surface of a connecting cylindrical part 7. Connecting cylindrical part 7 allows a hematocrit tube 9, which is an external medical device, to be connected. The hematocrit tube 9 to be used has a diameter larger than the outer diameter of connecting cylindrical part 7, the outer diameter including the height of projections 37. Hematocrit tube 9 and connecting cylindrical part 7 are connected such that connecting cylindrical part 7 is inserted into hematocrit tube 9. In this structure, because connecting cylindrical part 7 is provided with projections 37, hematocrit tube 9 can be connected firmly to connecting cylindrical part 7, which prevents hematocrit tube 9 from being accidentally dislodged.

Also, in this embodiment, because needle cap 3 (not shown) is not used to connect an external medical device to the needle assembly, needle cap 3 can be omitted from the needle assembly as appropriate. In such a case, the needle holding member 2 is configured so that fitting cylindrical part 5, which is a front part of needle holding member 2, is eliminated therefrom.

FIG. 21 shows a variation of this embodiment. In the embodiment shown in FIG. 21, hematocrit tube 9, which is an external medical device, is also provided with projections 38 on its inner surface in addition to projections 37 provided on the outer surface of connecting cylindrical part 7. Projections 38 are formed on the front part of hematocrit tube 9 to engage with the projections 37 formed on connecting cylindrical part 7. This allows hematocrit tube 9 to be connected firmly to connecting cylindrical part 7, which prevents hematocrit tube 9 from being accidentally dislodged.

Also, in this embodiment, because needle cap 3 is not used to connect an external medical device to the needle assembly, needle cap 3 can be omitted from the needle assembly as appropriate. In such a case, needle holding member 2 is configured so that fitting cylindrical part 5, which is a front part of needle holding member 2, is eliminated therefrom.

### Fourteenth Embodiment

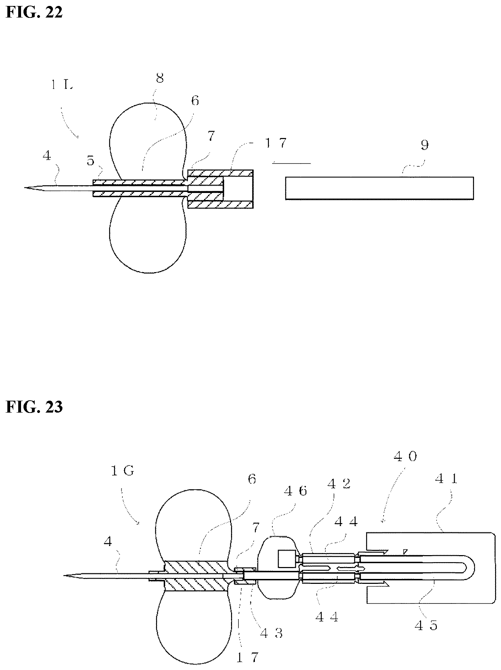

FIG. 22 shows a liquid collection-injection needle assembly 1L of the Fourteenth Embodiment of the present invention. In this liquid collection-injection needle assembly 1L, a connecting pipe 17 is connected to a connecting cylindrical part 7 of a needle holding member 2. Connecting pipe 17 is formed of a flexible material such as rubber, and has an inner diameter into which connecting cylindrical part 7 can be inserted. Connecting pipe 17 is connected to inserted connecting cylindrical part 7 by an adhesive. Connecting cylindrical part 7 and an external medical device are connected via this connecting pipe 17.

In this embodiment, the external medical device is a hematocrit tube 9. Hematocrit tube 9 is formed so as to have the size of an outer diameter, which allows tube 9 to be inserted into connecting pipe 17. The connection of hematocrit tube 9 to connecting cylindrical part 7 is performed by inserting hematocrit tube 9 into connecting pipe 17, which allows for communication between hematocrit tube 9 and connecting cylindrical part 7. This allows collection of blood or body fluids and injection of chemical solutions to be performed through a needle 4. In this embodiment, connecting cylindrical part 7 and hematocrit tube 9 are indirectly connected to each other via connecting pipe 17.

Also in this embodiment, because needle cap 3 (not shown) is not used to connect an external medical device to the needle assembly, needle cap 3 can be omitted from the needle assembly as appropriate. In such a case, needle holding member 2 is configured so that fitting cylindrical part 5, which is a front part of needle holding member 2, is eliminated therefrom.

### Fifteenth Embodiment

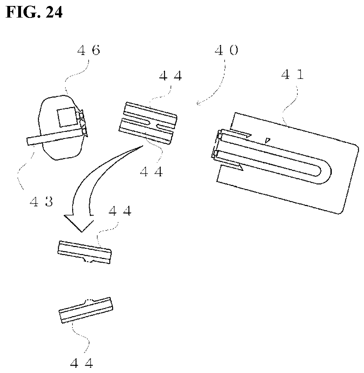

FIGS. 23 and 24 show a liquid collection-injection needle assembly 1G of the Fifteenth Embodiment of the present invention.

This embodiment provides an external medical device that uses an analyzer 40 shown in FIG. 23, which performs analysis of a slight amount of blood.

Analyzer 40 has a structure in which a capillary tube 42 is provided within a body part 41 formed of a transparent material. Capillary tube 42 is used to collect blood or body fluids. This capillary tube 42 consists of two tubes used for analysis 44 and a U-shaped tube 45, in which the two tubes used for analysis 44 communicate with each other via U-shaped tube 45. While U-shaped tube 45 is formed within body part 41, the two tubes used for analysis 44 are disposed outside body part 41. One end of tube for analysis 44 has an insert port 43, which is connected to a connecting cylindrical part 7 of liquid collection-injection needle assembly 1G.

The example of the needle assembly shown in FIG. 23 is the liquid collection-injection needle assembly 1G shown in FIG. 16. Capillary tube 42 is connected to needle assembly 1G such that insert port 43 is inserted into connecting cylindrical part 7, which allows blood or body fluids to be introduced into capillary tube 42. After capillary tube 42 has been filled with blood or body fluids, analyzer 40 is removed from liquid collection-injection needle assembly 1G, and then the tubes used for analysis 44 are separated from body part 41, so that the contents contained in tubes 44 are subjected to plasma analysis.

In this embodiment, connecting analyzer 40 directly to needle assembly 1G also allows analyzer 40 to be used for collection of blood or body fluids.

### Description of Reference Signs

1, 1A, 1B, 1C, ID, IE, IF, 1G, 1H, 1I, IJ, 1K, 1L liquid collection-injection needle assembly
2 needle holding member
3 needle cap
4 needle
5 fitting cylindrical part
6 holding cylindrical part
7 connecting cylindrical part
8 flap part
13 hematocrit tube
11, 12, 14, 15, 20, 25 projections
13 threaded member
17 connecting pipe
21 tapered surface
22 stepped surface
23 curved surface
26 pump member

## Claims

1. A liquid collection-injection needle assembly comprising: a needle, a needle holding member, and a needle cap;
the needle holding member comprising a holding cylindrical part that holds the needle after a needle base of the needle has been inserted thereinto, and a connecting cylindrical part that is formed so as to communicate with the holding cylindrical part,
the needle cap being formed into a tubular shape so as to cover an exposed portion of the needle, the exposed portion being exposed from the holding cylindrical part, and being configured to be detachably connected to the needle holding member;
wherein the needle cap is configured to be directly or indirectly connected to the connecting cylindrical part, so that the needle cap is capable of communicating with the connecting cylindrical part.

2. The liquid collection-injection needle assembly according to Claim 1, wherein the connecting cylindrical part and the needle cap form an engaging structure in which they are mutually engaged and detached.

3. The liquid collection-injection needle assembly according to Claim 1 or 2, wherein the needle cap is provided with a structure for connection that allows the needle cap being in a state connected to the connecting cylindrical part to be connected to an external medical device.

4. The liquid collection-injection needle assembly according to any of the preceding Claims, wherein the engaging structure is configured such that a front end of the needle cap is to be inserted into the connecting cylindrical part to be mutually fitted to each other so as to connect them.

5. The liquid collection-injection needle assembly according to any one of Claims 1 to 3, wherein the engaging structure is configured such that at least one of the connecting cylindrical part or an end portion of the needle cap is provided with projections that allow the connecting cylindrical part and the end portion of the needle cap to engage with each other.

6. The liquid collection-injection needle assembly according to any one of Claims 1 to 3, wherein the engaging structure is configured such that the connecting cylindrical part and the end portion of the needle cap are provided with a threaded member that allows the connecting cylindrical part and the end portion of the needle cap screw with each other.

7. The liquid collection-injection needle assembly according to any one of Claims 1 to 3, wherein the engaging structure is configured such that at least either of the connecting cylindrical part and the end portion of the needle cap is provided with a rough surface that generates frictional force between the connecting cylindrical part and the end portion of the needle cap.

8. The liquid collection-injection needle assembly according to any one of Claims 1 to 3, wherein the engaging structure is configured such that a tapered surface is formed on an inner surface of the needle cap, in which an inner diameter of the needle cap is continuously changed in an axial direction.

9. The liquid collection-injection needle assembly according to any one of Claims 1 to 3, wherein the engaging structure is configured such that a stepped surface is formed on the inner surface of the needle cap, in which the inner diameter of the needle cap is changed stepwise in the axial direction.

10. The liquid collection-injection needle assembly according to any one of Claims 1 to 3, wherein the engaging structure is configured such that the needle cap is formed of a flexible resin with a zigzag-shaped curved surface being formed on the inner surface thereof.

11. The liquid collection-injection needle assembly according to any of the preceding Claims, wherein the needle holding member is provided with a fitting cylindrical part to which the needle cap is detachably attached, the fitting cylindrical part communicating with the holding cylindrical part.

12. The liquid collection-injection needle assembly according to any of the preceding Claims, wherein a connecting pipe is connected to the connecting cylindrical part, and the end portion of the needle cap is connected to the connecting pipe.

13. A liquid collection-injection needle assembly comprising: a needle, and a needle holding member;
the needle holding member comprising a holding cylindrical part that holds the needle after a needle base of the needle has been inserted thereinto, and a connecting cylindrical part that is formed so as to communicate with the holding cylindrical part,
wherein the connecting cylindrical part is configured to be directly or indirectly connected to an external medical device.

14. The liquid collection-injection needle assembly according to any of the preceding Claims, wherein the holding cylindrical part is provided with a flap part that extends in the direction intersecting with the axial direction.

## Amended claims

### Amended claims under Art. 19.1 PCT

1. (Amended) A liquid collection-injection needle assembly comprising: a needle, a needle holding member, and a needle cap;
the needle holding member comprising a holding cylindrical part that holds the needle after a needle base of the needle has been inserted thereinto, and a connecting cylindrical part that is formed so as to communicate with the holding cylindrical part,
the needle cap being formed into a tubular shape so as to cover an exposed portion of the needle, the exposed portion being exposed from the holding cylindrical part, and being configured to be detachably connected to the needle holding member;
wherein the needle cap is configured to be directly or indirectly connected to the connecting cylindrical part, so that the needle cap is capable of communicating with the connecting cylindrical part, and
wherein the needle cap is provided with a structure for connection that allows the needle cap being in a state connected to the connecting cylindrical part to be connected to an external medical device.

2. The liquid collection-injection needle assembly according to Claim 1, wherein the connecting cylindrical part and the needle cap form an engaging structure in which they are mutually engaged and detached.

3. (Deleted)

4. (Amended) The liquid collection-injection needle assembly according to Claim 1 or 2, wherein the engaging structure is configured such that a front end of the needle cap is to be inserted into the connecting cylindrical part to be mutually fitted to each other so as to connect them.

5. (Amended) The liquid collection-injection needle assembly according to Claims 1 or 2, wherein the engaging structure is configured such that at least one of the connecting cylindrical part or an end portion of the needle cap is provided with projections that allow the connecting cylindrical part and the end portion of the needle cap to engage with each other.

6. (Amended) The liquid collection-injection needle assembly according to Claim 1 or 2, wherein the engaging structure is configured such that the connecting cylindrical part and the end portion of the needle cap are provided with a threaded member that allows the connecting cylindrical part and the end portion of the needle cap screw with each other.

7. (Amended) The liquid collection-injection needle assembly according to Claim 1 or 2, wherein the engaging structure is configured such that at least either of the connecting cylindrical part and the end portion of the needle cap is provided with a rough surface that generates frictional force between the connecting cylindrical part and the end portion of the needle cap.

8. (Amended) The liquid collection-injection needle assembly according to Claim 1 or 2, wherein the engaging structure is configured such that a tapered surface is formed on an inner surface of the needle cap, in which an inner diameter of the needle cap is continuously changed in an axial direction.

9. (Amended) The liquid collection-injection needle assembly according to Claim 1 or 2, wherein the engaging structure is configured such that a stepped surface is formed on the inner surface of the needle cap, in which the inner diameter of the needle cap is changed stepwise in the axial direction.

10. (Amended) The liquid collection-injection needle assembly according to Claim 1 or 2, wherein the engaging structure is configured such that the needle cap is formed of a flexible resin with a zigzag-shaped curved surface being formed on the inner surface thereof.

11. (Amended) The liquid collection-injection needle assembly according to any of the preceding Claims except for Claim 3, wherein the needle holding member is provided with a fitting cylindrical part to which the needle cap is detachably attached, the fitting cylindrical part communicating with the holding cylindrical part.

12. (Amended) The liquid collection-injection needle assembly according to any of the preceding Claims except for Claim 3, wherein a connecting pipe is connected to the connecting cylindrical part, and the end portion of the needle cap is connected to the connecting pipe.

13. (Deleted)

14. (Amended) The liquid collection-injection needle assembly according to any one of Claims 1 to 12 except for Claim 3, wherein the holding cylindrical part is provided with a flap part that extends in the direction intersecting with the axial direction.
